(19) **Europäisches Patentamt**

**European Patent Office**

**Office européen des brevets**

(11) **EP 1 674 119 A1**

(12) **EUROPEAN PATENT APPLICATION**

(43) Date of publication:
   **28.06.2006 Bulletin 2006/26**

(51) Int Cl.:
   *A61M 1/10* (2006.01)

(21) Application number: **04425940.6**

(22) Date of filing: **22.12.2004**

(84) Designated Contracting States:
   **AT BE BG CH CY CZ DE DK EE ES FI FR GB GR HU IE IS IT LI LT LU MC NL PL PT RO SE SI SK TR**
   Designated Extension States:
   **AL BA HR LV MK YU**

(71) Applicant: **Tecnobiomedica S.p.A.**
   **00040 Pomezia (RM) (IT)**

(72) Inventors:
   • **Rinaldi, Stefano**
   **c/o Tecnobiomedica S.P.A.**
   **00040 Pomezia (IT)**

   • **Zagara, Maurizio**
   **c/o Tecnobiomedica S.P.A.**
   **00040 Pomezia (IT)**
   • **Carotenuto, Luigi**
   **c/o Tecnobiomedica S.P.A.**
   **00040 Pomezia (IT)**

(74) Representative: **Bosotti, Luciano**
   **c/o Buzzi, Notaro & Antonielli d'Oulx Srl,**
   **Via Maria Vittoria, 18**
   **10123 Torino (IT)**

(54) **A pulsator device, method of operating the same, corresponding system and computer program**

(57) Described herein is a pulsator device (1) that can be used for generating a pulsed flow starting from a substantially constant flow, for example in reactors for cell growth and other applications in which it is desired to have available pulsed irroration flows. The device comprises a deformable body that is able to define a duct (10) for passage of a substantially constant flow ($Q_0$) of a fluid subjected to pumping. Associated to the deformable body (10) is at least one actuation chamber (14) that is selectively expandable between at least one contracted condition and at least one extended condition of pumping so as to produce a variation of the section for passage of said fluid through the duct (10). The variation of the section of passage through the duct (10) is able to cause the generation of a pulsed flow ($Q(t)$) of the fluid subjected to pumping.

Fig. 1

## Description

Field of the invention

The present invention relates to pulsator devices that can be used, for example, in systems for cardiac assistance, such as extracorporeal circulation (ECC) systems designed to ensure the continuity of the function of pumping and oxygenation of the blood during open-heart operations, or in devices for cardiopulmonary assistance, commonly referred to as ECMO (ExtraCorporeal Membrane Oxygenation) devices and the like.

In any case, the advantage of having available pulsed flows is appreciated also in other sectors of the biomedical industry, for example, in the sector of tissue engineering. It has in fact been possible to verify that cell structures developed in bio-reactors operating in conditions of pulsed flow and pulsed pressure enable the production of cardiovascular prostheses that are as a whole better than the ones that are obtained in stationary conditions (constant pressures and flows).

Description of the related art

In systems for extracorporeal circulation of a traditional type, the pumping action is performed using pumping elements (such as, for example, centrifugal pumps or peristaltic pumps), which can generate a substantially constant flow.

In more recent times, it has been found that the possibility of providing a pulsed flow, like the one produced by the natural heart, is advantageous from numerous standpoints, precisely because it is able to reproduce with greater faithfulness the conditions of operation of the natural cardiovascular system.

Above all in applications in systems for extracorporeal circulation and in devices for cardiac assistance, it is important that the operation of the pulsator should be controllable in a precise and reliable way in relation to different parameters such as, for example, the flow rate of the pulsator device, the (absolute and relative) duration of the phases of systole and diastole and, in some cases, the possible synchronization with the operation of the natural cardiac muscle.

For a review of said problems useful reference may be made to WO-A-01/43797.

In producing pulsator devices of the type considered previously it is necessary to take into account various factors.

For example, at least in some applications, said devices must be connectable in the vicinity of the natural heart of the carrier.

Furthermore, it is desirable that the devices should be structurally simple, with shapes such as not to give rise to problems of stagnation of blood flow, with adverse effects, such as the formation of thrombi and/or emboli, which can derive from said phenomenon.

In addition, the devices in question must preferentially present characteristics that will enable their production with materials that have affirmed their validity and have been widely experimented in the biomedical field, also as regards the possibility of said materials to undergo sterilization and surface treatments, such as, for example, the ones used for improving biocompatiblity and haemocompatiblity.

It is then important that the devices, particularly in their single-use parts, i.e., ones that cannot be reused, can be produced in a simple and inexpensive way.

As regards the modalities of operation, it is desirable that the pulsator device should be controllable so as to reproduce faithfully - taken in itself or in co-operation with the elements to which it can be associated (including the natural heart of the carrier) - the operation of the natural cardiovascular system. All this must be obtained in a simple and reliable way, reducing to the minimum the parameters that must be measured to be able to ensure correct operation of the device.

The general aim is to render the blood flow in the aorta pulsed (i.e., physiological) in the course of ECC or of percutaneous cardiopulmonary (or paracorporeal cardiocirculatory) assistance. This can be obtained using ECC primary pumps, normally set upstream of the gas-exchanger device (oxygenator), of a pulsed type (or peristaltic pumps rotating at a modulated speed), but said solution entails various disadvantages, amongst which the main ones are: the need to operate at very high pressures on account of the significant fluid resistance of the oxygenators; very high peak and instantaneous rates of the blood in the oxygenator, with considerable shear stresses and risk of haemolysis; damping of the pulsatility as a result of the elastance of the oxygenator and of the blood line; etc. The solution proposed intends, instead, to achieve the aim, leaving as primary pump of the ECC the one normally used (a peristaltic or centrifugal continuous-flow pump) and adding, downstream of the oxygenator and of the possible arterial filter, a device that is able to modulate the flow, rendering it physiologically pulsed.

Similar approaches have already been more than once taken into consideration, without, however, finding use in the common practice on account of problems of complexity, cost and thrombogenicity. Said problems derived mainly from the choice of using pulsed pumps, with the consequent need to have a reservoir (equivalent to the atria of the heart), which is able to accumulate the incoming blood during the systole phase of the pump, and of using (passive or active) valves for guaranteeing unidirectionality of the flow.

The alternative to the use of a pump consists in employing a flow modulator (the so-called "pulsator"), i.e., an element with variable volume, which receives, at its inlet, the incoming flow ($Q_0$), which is continuous or practically continuous, and delivers at outlet a flow (Q) equal to the inlet flow decreased or increased by its variation in volume.

The main requirements that a flow modulator must satisfy in order to find practical application in ECC systems or systems of percutaneous cardiopulmonary (or paracorporeal cardiocirculatory) assistance or in other fluid-circulation systems that call for similar characteristics, such as for example those of bioreactors, are:

- simplicity of construction and economy both of the system of actuation and control of the pulsator and, in particular, of the pulsator itself and of the parts connected thereto that must be replaced at each use of the system (single-use components);
- safety and reliability;
- extremely low thrombogenicity (or risk of formation of deposits in the case of liquids other than the blood) to be guaranteed not only with the use of adequate materials but also, and principally, by means of a geometry of the pulsator that enables the blood (or other liquid) to flow without forming areas of stagnation or turbulence and in such a way as to guarantee a good washing of the internal surfaces of the device;
- applicability to ECC systems (or to systems for cardiopulmonary assistance or cardiocirculatory assistance, or to other systems for the circulation of liquids) of a different type and/or supplied by different manufacturers, without requiring interconnections with or modifications of these systems; and
- possibility of switching rapidly and simply between the condition of pulsed flow and that of continuous flow.

All these requirements are not adequately met by the known solutions.

Purpose and summary of the invention

The purpose of the present invention is to provide an altogether satisfactory response to the needs and requirements set forth above, needs and requirements which, as has been seen, can prove at least in part even in contrast with one another.

According to the present invention, said purpose is achieved thanks to a pulsator device having the characteristics referred to specifically in the ensuing claims. The invention also relates to a corresponding method of operation, a corresponding system, as well as to a computer-program product which can be loaded into the memory of at least one computer and comprises portions of software code for implementing the method according to the invention. The claims form an integral part of the teachings regarding the invention provided in the present patent application.

Brief description of the annexed plate of drawings

The invention will now be described, purely by way of non-limiting example, with reference to the annexed plate of drawings, in which:

- Figure 1 is a block diagram representing a flow modulator incorporating a pulsator device of the type described herein;
- Figure 2 is an overall view in longitudinal cross section of a pulsator device of the type described herein;
- Figure 3 is a cross-sectional view according to the line III-III of Figure 2;
- Figure 4 is a diagram representing some physical quantities, to which reference is made in the description;
- Figure 5 comprises two parts, designated respectively by a) and b), consisting of diagrams illustrating the working principle of a pulsator device of the type described herein;
- Figures 6 and 7 also each comprise two parts designated respectively by a) and b), consisting of diagrams representing various modalities of actuation of a pulsator device of the type described herein; and
- Figure 8 is the diagram of a possible mode of synchronization of the actuation of the pulsator device with an external reference signal, such as an electrocardiograph signal (ECG).

Detailed description of examples of embodiment of the invention

In the figures of the annexed plate of drawings, the reference number 1 designates as a whole a pulsator device that can be used, for example, in ECC systems, in devices for cardiac assistance, or in biological reactors, in accordance with the criteria to which general reference has already been made in the introductory part of the present description, also considering WO-A-01/43797.

The device 1 illustrated herein is configured as a tubular duct which is to be traversed by a flow of liquid, such as a biological liquid.

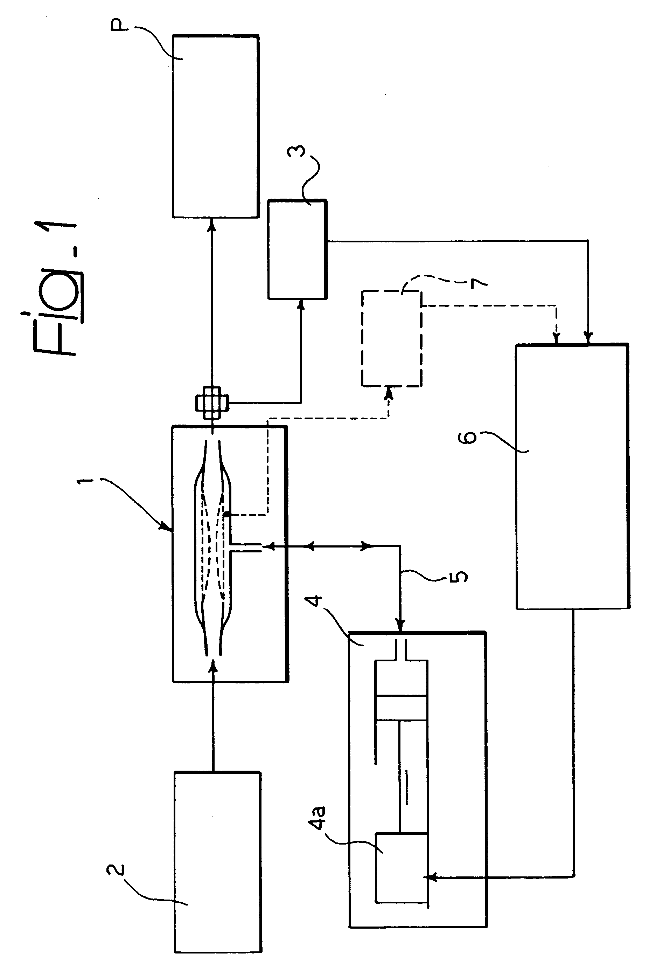

In the sequel of the present description, it will be assumed (without this, however, implying that it should be interpreted as in any way limiting the scope of the invention), that said fluid is blood and that the flow through the respective pulsator 1 occurs, with reference to the point of observation of Figure 1, from left to right.

Specifically, the block diagram of Figure 1 represents a flow modulator that uses a pulsator device 1, which, thanks to its variable volume, receives a continuous flow coming from an ECC system 2 (or other system) and sends a pulsed flow to the patient (or other load), designated as a whole by P.

The pulsator 1 basically consists of a duct 10, usually fundamentally inextensible but flexible, contained in a shell 12, which is substantially rigid and equipped with connectors for inlet 12a and outlet 12b. The gap between the shell 12 and the duct 10 constitutes a closed space or volume, also around the connectors 12a and 12b, but equipped with a connector which enables controlled introduction or removal of a fluid (the so called "actuation fluid"), which acts with variable and controllable pressure on the flexible duct 10 bringing about the desired variations of volume.

Set at output from the pulsator 1 is the sensor of a flowmeter 3 for measuring the blood flow (or other flow) delivered to the patient P. The flowmeter 3 can be of the ultrasound type and hence based upon a sensor without contact with the blood and which can be used a large number of times.

In the example of embodiment illustrated, the flowmeter 3 is the only measuring instrument/sensor required, thanks to the original control logic which will be described hereinafter, for adequate operation of the flow modulator.

A pump 4, driven by a motor 4a, such as for example a linear motor, enables the actuation fluid to be pushed into or removed from the gap of the pulsator, i.e., it enables an actuation fluid to be transferred in a controlled way with respect to the pulsator 1. This occurs via a closed fluid circuit 5 connected on one side to the pump 4 and on the other to the gap (or actuation chamber) of the pulsator 1 and containing the actuation fluid.

A control unit 6 receives the signal of the flowmeter 3 (and other possible optional sensors) and controls the action of the pump 4 issuing a command to its motor 4a on the basis of the logics described in what follows.

Optionally, other components of a known type can prove useful for improving the performance and safety of the system.

One of these may, for example, be a pressure meter 7 with the sensor set in the gap of the pulsator 1 or in any other position of the closed circuit 5 containing the actuation fluid. This meter, indicated by dashed lines in the diagram of Figure 1, makes available a redundancy of information useful for the purposes of safety and simplicity of the control. Also this, however, is not in contact with the blood and can be repeatedly used.

Another optional component (not illustrated explicitly in the plate of drawings) is a system for monitoring of the volume of actuation fluid contained in the circuit 5 and for compensation of possible small leakages of fluid which may arise, for example, in the connections. Said systems are widely known and used, for example, in intra-aortic-counterpulsation systems and in the ventricular assist devices (VADs) of a pneumatic type.

As has already been said, the pulsator 1 is basically constituted by a flexible duct 10 contained in a rigid shell 12 and equipped with connectors for inlet 12a and outlet 12b. One of the important requirements of this component is the low thrombogenicity. For this purpose, the solution described herein causes the blood flow within the pulsator to be as regular as possible, without any stagnation or turbulence, and the internal surface to be well "washed" by the flow to prevent any formation of deposits that may grow to significant dimensions and subsequently detach, forming emboli.

To meet this requirement, a geometry of the duct optimized from the fluid-dynamics standpoint is adopted like the ones known in the art for different types of pumps for blood, in particular for VADs and for different models of artificial heart.

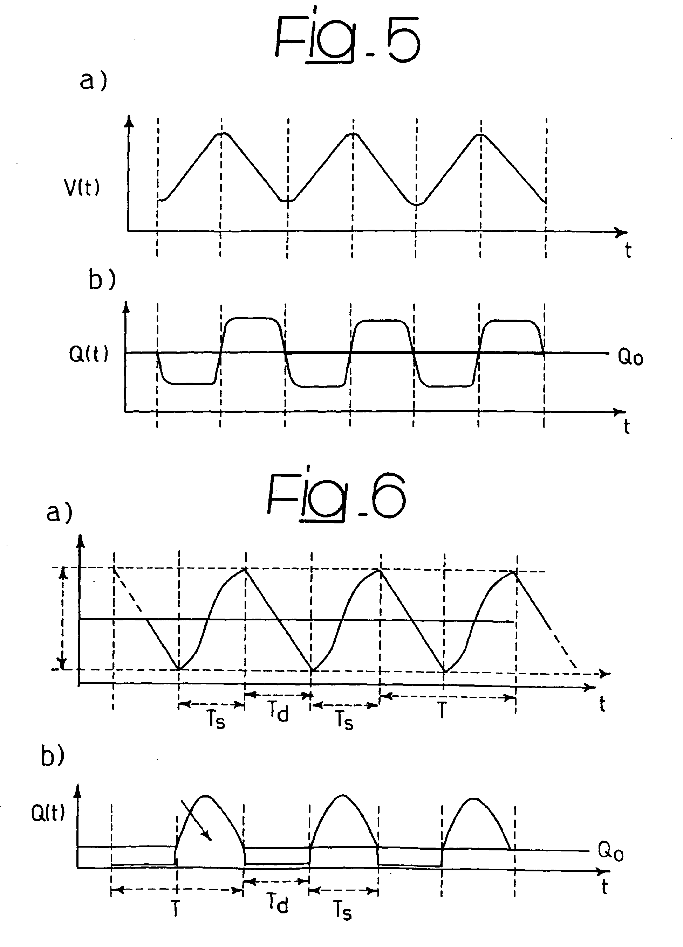

For the present application, it proves particularly advantageous to use a flexible duct 10 having the configuration of a straight tube contained in a shell, which is also cylindrical and longitudinally anchored thereto along three directrices at 120°, as may be seen more clearly in the cross sectional view of Figure 3.

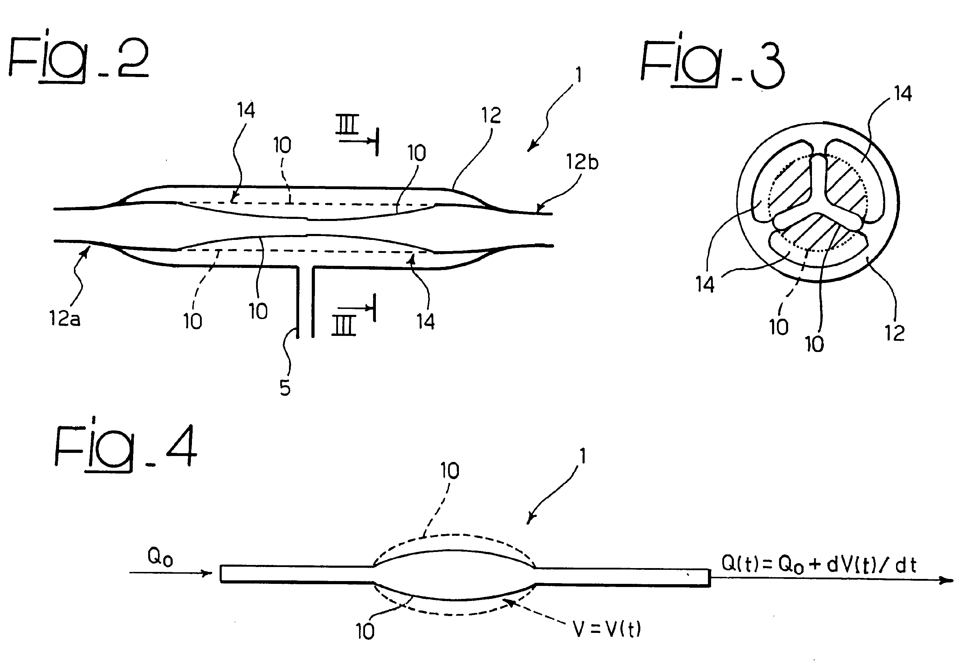

An advantage of this configuration is that within it the flexible duct 10 can be long and have a relatively small diameter and thus be sized so as to form, in the completely expanded condition of the duct (illustrated with a dashed line in Figures 2 and 3), a continuous tube with the ones that connect the ECC system with the patient P, thus guaranteeing optimal fluid-dynamics and washing of the surfaces. Furthermore, the bends that the duct forms in the course of its deformation towards the condition of complete contraction of the duct (illustrated by a solid line in Figures 2 and 3) remain basically aligned to the direction of the flow and thus enable maintenance of an effective washing of the surfaces also during the phases of deformation.

The disposition at 120° of the lines of anchorage of the flexible duct 10 to the rigid shell 12 currently emerges as the preferred solution to arrive at a state of complete contraction characterized by a minimal but non-zero residual cross section. This enables minimization of the overall dimensions of the system (for a pre-set maximum ejection volume), preventing, however, possible problems of injury to the blood (haemolysis) which could arise if the internal surfaces of the flexible duct were to adhere owing to collapse.

In the currently preferred embodiment, the duct 10 is made as a tubular body of deformable material, for example silicone rubber or polyvinyl-chloride (PVC), nylon, pebax or polyurethane, compatible with the uses in the medical field, surrounded by the body 12, which is also made preferentially of plastic material, such as for example polycarbonate, polysulphone or PVC, with a wall having an as a whole cylindrical shape, gradually tapered in a position corresponding to the connectors 12a and 12b.

The cavities 14, i.e., the chambers for operation of the device in which the actuation fluid is introduced thus extend (with possible not markedly appreciable discontinuities) throughout the axial length of the pulsator device 1.

In the example of embodiment illustrated herein, corresponding to a currently preferred embodiment, the cavities or actuation chambers 14 are thus three in number, possibly interconnected. Each actuation chamber assumes - in resting conditions - a general tile shape with an angular extension (referred to the principal central axis of the duct 10) of a little less than 120°.

The structure just described is suited to being made with the use of plastic materials, such as the ones cited previously resorting to normal techniques of extrusion and/or moulding. These are techniques widely experimented in the biomedical sector, as documented, for example, by EP-A-0 512 359, regarding the fabrication of balloon catheters.

Another important characteristic of the pulsator device 1 illustrated herein is that it is a non-valved device, i.e., one in which there are not present valve elements designed to regulate, by opening and closing, the flow through the pulsator 1. As will be seen more clearly from what follows, in fact, in the system described herein the pulsed character of the outgoing flow is obtained only by controlling the operation of the pulsator.

Via the duct 5, the pumping element 4 causes a pressurized fluid to flow into the cavities 14 in such a way as to cause the chambers defined by the cavities 14 to be able to swell, as a result of the as a whole flexible character of the material constituting the duct 10 of the pulsator device 1, so as to be deformed towards the condition of maximum deformation of the cavities represented with solid line in Figures 2 and 3.

In said condition, the chambers corresponding to the cavities 14 swell, and the portion of wall comprised between each of these chambers and the duct 10 of the pulsator device 1 becomes arched, developing a more or less marked convexity directed towards the duct 10.

This movement of expansion and arching (which occurs in a substantially identical way for all three cavities 14), with consequent contraction of the tubular duct 10 of the pulsator device 1, underlies a clear phenomenon of modulation of the outgoing flow.

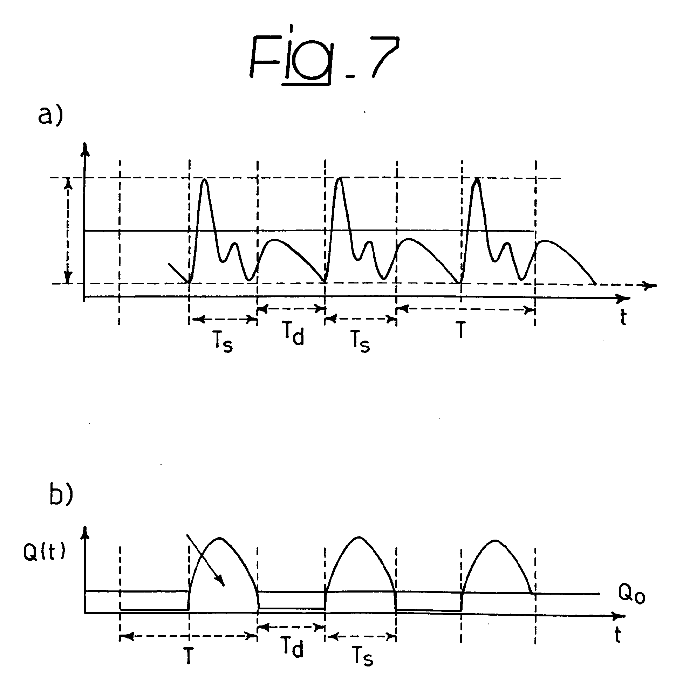

As is illustrated more clearly in a schematic form in the diagram of Figure 4, the expansion - and, in a dual way, the contraction - of the chambers 14, controlled via the pump 4, is able to cause in time a variation of the volume $V = V(t)$ of the duct 10 of the pulsator. Said variation enables a constant or substantially constant blood flow $Q_0$, which is sent at inlet to the pulsator device 1 (on the left-hand side of the device 1 - as illustrated in Figure 4), to be converted at outlet from the device 1 (right-hand side of Figure 1) into a pulsating flow $Q(t) = Q_0 + dV(t)/d(t)$.

The fact that previously mention was made of a "substantially" continuous flow rate at inlet to the pulsator aims at taking into account the fact that the continuous flow at outlet from certain pumping devices, such as for example the peristaltic pumps, may in fact present reduced fluctuations due to "ripple" phenomena (linked to the intrinsic mechanism of the pumping action) or resulting from variations in the pressure downstream.

Figure 5 b) represents, against the background of a corresponding incoming flow $Q_0$, a possible time evolution of a pulsating outgoing flow $Q(t)$ determined by variations in the internal volume of the pulsator, such as the ones represented schematically in Figure 5 a). The pulsating flow $Q(t)$ is distinguished by a period T (variable in time), in which there is distinguishable a diastole phase $T_d$, during which the volume of the duct 10 increases or remains constant and consequently with a value of the flow $Q(t)$ lower than or at the most equal to the incoming flow $Q_0$, and a systole phase $T_s$, in which the volume of the duct 10 decreases and, consequently, the flow $Q(t)$ increases gradually towards a maximum value, to return then to the reduced value at a point corresponding to the start of the subsequent diastole phase.

For simplicity of illustration, the diagram of Figure 5 a) illustrates a possible variation in the volume of the duct 10 according to a rounded-triangular-wave law, whilst the diagram of Figure 5 b) shows a possible corresponding modulation of the outgoing flow in the form of a square wave with rounded leading and trailing edges.

A system of the type illustrated in Figure 1 is used, being interposed along the line that takes the blood from the outlet of the ECC system or the system providing assistance to the patient. Otherwise, the modalities of application and connection of a system of the type illustrated in Figure 1 correspond - both as regards the surgical method of connection to the patient and as regards the accessory devices required to make such a connection - to data, information, and criteria to be deemed in themselves known in the art and, in any case, in themselves not important for an understanding and implementation of the invention.

Passing now to an even more detailed examination of the modalities of use of a system such as the one illustrated in Figure 1, it should be recalled that, in order to meet as well as possible the requisite of low thrombogenicity, it is appropriate for the duct to reach at each cycle, at the end of the diastole phase $T_d$, the position of complete expansion, thus assuming the configuration of a tube and hence enabling an optimal washing of its internal surface. This need can be met by intervening on the actuation and control unit 6.

Passing now to an examination of various possible modalities and logics of actuation of the pulsator 1, it will be noted that the deformation of the flexible duct 10 of the pulsator 1 occurs as a result of the difference in pressure between the blood inside it and the actuation fluid contained in the gaps (chambers 14) of the pulsator 1. The pressure and volume of this actuation fluid is varied by operating the pump 4, which has precisely the function of transferring, in a controlled way (by the unit 6), an actuation fluid with respect to the actuation chambers 14 of the pulsator 1.

The actuation fluid can be either a liquid or a gas. The use of a liquid, given its incompressibility, enables a more direct and immediate actuation and control (volumetric control). It involves, however, a greater weight and, principally, gives rise to reasons of possible critical aspects linked to factors of sterizability, packaging and transportation. The use of a gas such as air or, rather, $CO_2$ or helium imposes the need for a pressure control, which is less direct. This is, on the other hand, feasible using known technologies, such as for example the ones used for actuation of intra-aortic counterpulsators.

In general, actuation and control of the pulsator 1 start with a diastole phase, during which the control unit 6 measures, via the flowmeter 3, the flow Q(t) coming out of the duct 10 and imposes, by controlling the pump 4 during intake, the need for it to be approximately zero (or only slightly positive).

This action can be performed using a normal control logic, for example of a PID type, up to complete filling of the pulsator 1 (duct 10 completely expanded). Once said state of complete expansion and filling of the duct 10 has been reached, notwithstanding the action of suction of the pump, the duct 10, which is flexible but inextensible, will no longer be able to increase its own volume and accumulate blood. The flow $Q_0$ entering the pulsator will hence traverse it and come out to reach the patient P.

The flowmeter 3 will, at this point, indicate a relatively sharp increase in the flow rate, which the control unit 6 will be able to recognize (either as it stands or as a difference between the desired flow rate and the measured flow rate) as end of the phase of filling of the duct 10, accordingly issuing a command for arrest of the action of suction of the pump and enabling the start of the systole phase, which can be immediate or deferred when it is desired that the systole should start at instants determined by other conditions, such as for example the synchronism in counterpulsation with the natural heart (in the terms described in greater detail in what follows).

When the state of complete filling of the pulsator 1 is reached, this can be detected by the control unit 6 also by monitoring the current for actuation of the motor 4a of the pump and/or (if the corresponding sensor is available) the pressure of the actuation fluid. The former, in fact, on account of the unbalancing between the value of flow imposed and the value measured and on account of the of the inextensiblity of the tubular duct 10, will have a sharp increase. The latter, instead, on account of the action of suction of the pump 4, not compensated by the increase of volume of the flexible duct, will undergo a sharp drop.

Said different modalities of detection of reaching the state of complete filling of the pulsator 1 can be used as an alternative or for the purpose of having a redundancy of information in order to greater safety.

The control logic of the modulator in the phases of systole depends then upon the modalities of actuation that the user may wish to adopt.

The tests so far conducted by the inventors have led to an identification of three basic operating modes, namely:

- asynchronous pulsation with fixed ejection volume;
- asynchronous pulsation at a predetermined frequency; and
- pulsation synchronous with the heart beat (or other reference signal).

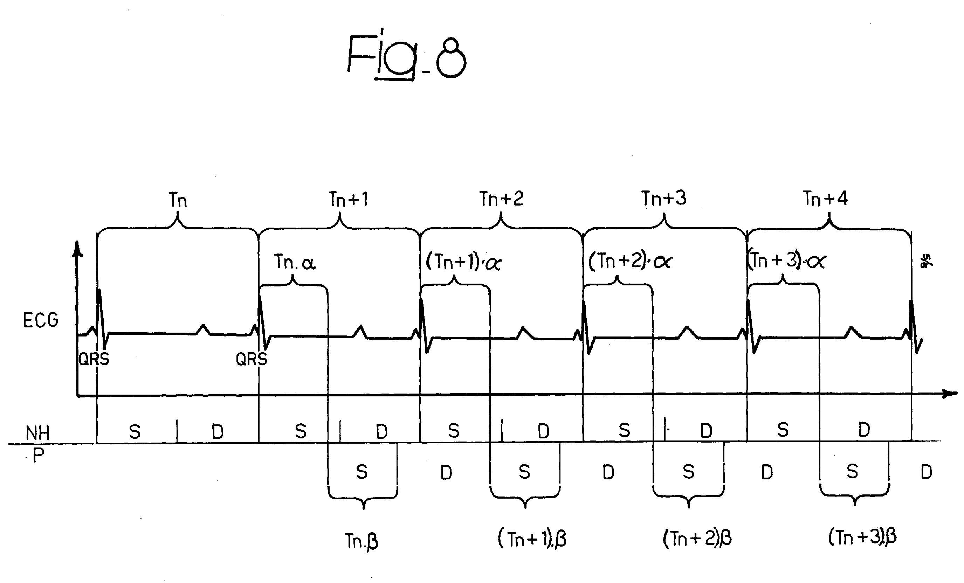

Said different possible modes of operation will now be described with reference to Figures 6 and 7 of the annexed plate of drawings. Each of said figures comprises two portions, designated, respectively, by a) and b).

Of these, the portion b) reproduces, according to modalities altogether similar to the ones adopted in Figure 5 b), the time evolution of the pulsating flow Q(t) at outlet from the pulsator 1 with direct reference to the incoming flow $Q_0$, which is substantially constant.

The portion designated by a) is instead a diagram representing the pumping action exerted by the pump 4. Supposing that the pump in question is a piston pump, the diagrams a) of Figures 6 and 7 can be simply viewed as representing the position assumed in time by the piston of the pump itself. The minimum value of the corresponding curve represents the position of end of diastole, with the pulsator 1 "full fill".

Of course, this representation can be transposed in an elementary way to pumps of different types.

For the operating mode with pulsation synchronous with the heart beat, reference will moreover be made to Figure 8, which contains a diagram exemplifying the synchronization between the external reference signal (ECG) and the phases of systole and diastole in the actuation of the pulsator.

Asynchronous pulsation with fixed ejection volume

This operating mode, to which Figures 6 and 7 refer, is typically the one that can be used for improving, thanks to the pulsatility of the flow and of the pressure, the perfusion of the organs in the course of heart-surgery operations of long duration conducted in extracorporeal circulation.

In this operating mode the aim is to have at each cycle (period T) the ejection volume to the patient corresponding to an adjustable pre-set value. Of course, this may not: be higher than the maximum volumetric range of the pulsator 1 plus the incoming mean flow multiplied by the duration of the systole phase $T_s$.

The systole phase starts, in this case, immediately after the complete filling of the pulsator and consists in operating the pump 4 in ejection with a pulse of pre-set form but with controlled amplitude and duration in such a way as to cause an ejection volume per cycle equal to the pre-set one.

In the case where, as actuation fluid, a liquid is used, the variations in the internal volume of the pulsator correspond exactly to the volume displaced by the motion of the piston of the pump. In this case, a motion in ejection is imposed on the piston of the pump, starting from the position reached at the end of the diastole, which has a pre-set time evolution such as to generate an adequate waveform of the output flow rate. The amplitude of the motion is continuously adjusted by the control unit in such a way as to cause an ejection volume, determined by integrating the signal of the flowmeter 3 in the systole phase, equal to the one set, thus compensating for possible variations of the incoming flow $Q_0$.

The duration of the systole phase can then be varied to keep it proportional (for example equal) to the duration of the diastole $T_d$. For this purpose, since the value of the incoming flow $Q_0$ is not known *a priori,* the control unit 6 corrects, at each cycle, the duration of the movement of compression, adapting it to the duration of the preceding diastole, which is in turn determined by the ejection volume imposed and by the incoming flow. Once the systole phase terminates, a subsequent new diastole phase starts immediately.

If we define:

VE = ejection volume (per cycle)
$\Delta V$ = variation in volume of the pulsator
$\Delta V_{max}$ = maximum variation of the volume of the pulsator
$\Delta V_{opt}$ = optimal variation of the volume of the pulsator
$Q_0$ = incoming flow
$Q(t)$ = outgoing flow
$T_d$ = duration of the diastole phase
$T_s$ = duration of the systole phase

the simple relations that correlate the various quantities are:

$$VE \ = \ Q_0 \ \times \ T_s \ + \ \Delta V$$

$$T_d \ = \ \Delta V \ / \ Q_0 \ -> \ Q_0 \ = \ \Delta V \ / \ T_d$$

By then setting

$$T_s \ = \ k \ \times \ T_d \ (\text{for example } k \text{ equal to } 1)$$

we have:

$$VE \ = \ k \ \times \ T_d \times \Delta V \ / \ T_d \ + \ \Delta V \ = \ \Delta V \ \times \ (1 \ + \ k)$$

$$\Delta V \ = \ VE \ / \ (1 \ + \ k)$$

$$T_d \ = \ VE \ / \ ((1 \ + \ k) \ \times Q_0)$$

This operating mode with liquid as actuating fluid is illustrated in Figure 6.

In summary, in this operating mode, the incoming flow is imposed from the outside, the ejection volume and the ratio between the duration of the systole and that of the diastole are fixed, and the system automatically adapts in a simple way the amplitude and duration of the motion in compression of the piston to generate in the desired period of time a reduction in the volume of the pulsator such as to generate the ejection volume set.

Normally, when operating with fixed ejection volume, the endeavour is to get the pulsator 1 to work with a volumetric range close to the maximum (condition defined as "full fill - full empty"), i.e., with a range ($\Delta V_{opt}$) close to the maximum range allowed by the duct 10 between a condition of maximum extension and a condition of maximum contraction.

This result can be achieved by adjusting manually the regulation of the ejection volume set or, rather, by adding to the control unit 6 (operating according to criteria in themselves known) an external and slow feedback loop based upon the comparison between the effective values of $\Delta V$ and $\Delta V_{opt}$.

In the case where a gas is adopted as actuation fluid, the control logic is altogether similar, it being, however, necessary to take into account the effects resulting from the compressibility of the gas and the inertance of the masses of liquid (blood) that the pulsator must set in motion. The latter determines the need for a high pressure in the gap (chambers 14) of the pulsator 1 at start of systole, in order to set the blood in motion towards the patient, and for a marked reduction in the pressure itself at the end of systole in order to slow down its motion.

For the above reason, it may be expedient to use, for the systole phase, a control acting on the pressure in the actuation fluid, measured preferably in the gap of the pulsator or close thereto, instead of on the position of the piston.

Also in this case, one imposes to the piston of the pump 4 an actuation (stroke or generated pressure) based on a predetermined waveform having as adjustable variables the amplitude and the duration of the action of compression exerted by the pump. The adjustment of these variables is made by the control unit 6 on the basis of a logic altogether similar to that of the previous case: the amplitude is varied to maintain the integral of the outgoing flow during the systole phase equal to the desired ejection volume, and the duration is modified to keep it proportional to the duration of the diastole.

The waveform to be used in this case for the motion of the piston or for the pressure of the actuation fluid (gas) can be optimized on the basis of experimental tests and qualitatively has, on the basis of the tests conducted, the form indicated in the diagram of Figure 7.

In summary, in the asynchronous operating mode with predetermined ejection volume, the system operates, to maintain the outgoing flow zero, by drawing actuation fluid from the chambers 14 of the pulsator 1 until complete filling of the tubular duct and then by compressing the actuation fluid in the chambers with an action of amplitude such as to eject the desired volume of blood. The frequency of actuation, and hence the duration of the diastole and systole phases, are automatically determined and vary as the mean flow varies, said mean flow being set by the ECC system, and as the ejection volume, which is set, varies.

Asynchronous pulsation at a predetermined frequency

If we wish to maintain the frequency of pulsation equal to a pre-set value, and hence with a predefined duration of the systole and diastole phases, it is possible to control the system exactly as illustrated for the previous operating mode but varying the ejection volume, and hence the amplitude of the actuation in the systole phase, in such a way as to maintain the desired frequency.

Of course, the pulsator 1 will no longer operate in an "full fill - full empty" way but in an "full fill-partially empty" way, i.e., with a range comprised between a condition of maximum extension and a condition of contraction that is only partial. The frequency set must be higher than what it would be in the case of operation in the mode with ejection volume mode fixed and optimized in order to ensure the "full fill- full empty" condition.

This operating mode is of practical interest when, using the previous mode, there would be an excessively low frequency of pulsation on account of the low mean flow rate with respect to the maximum volumetric range of the pulsator 1. This is the case, for example, of application of the system with patients in the paediatric age range or in any case small patients.

Also in this case, the systole phase starts immediately after complete filling of the pulsator and consists in operating the pump 4 in ejection with a pulse of predetermined duration in relation to the frequency of actuation desired and of amplitude initially equal to a predefined standard value. Immediately after this systole phase a diastole phase starts, which is made to proceed up to complete filling of the duct 10, detected as described previously. The time necessary to reach this condition is then compared with the desired duration of the diastole as a result of the desired frequency of actuation. If said time is longer or shorter than desired, the approach is to reduce or increase the amplitude of the action of actuation in the systole phase progressively so as to reduce or increase, respectively, the ejection volume VE and accordingly shorten or lengthen, respectively, the duration of the diastole. This progressive operation of control proceeds until a duration of the diastole, and hence a frequency of actuation, equal to the value desired and set is obtained and maintained.

Pulsation synchronous with the heart beat

This operating mode is of interest when the system is used with patients whose heart beats regularly but does not have the force to sustain adequately the circulation of the blood and hence guarantee an adequate perfusion of the organs. Typical examples of this situation are the so-called "post-cardiotomy assistance", "percutaneous cardiopulmonary assistance" or "extracorporeal membrane oxygenation". In the first case, they are patients subjected to heart-surgery operations in extracorporeal circulation whose heart, at the end of the intervention, does not recover an adequate functionality. In these cases, it is necessary to keep the extracorporeal circulation (or any other procedure of assistance) active for long periods of time (some hours or a few days) in the hope that the heart will recover from the trauma of the operation and will recover its own functionality. Percutaneous assistance or ECMO are instead used in patients with serious cardiac insufficiency consequent to pathological conditions, usually of an acute nature (e.g., post-infarct shock), in order to guarantee an adequate perfusion of the organs whilst awaiting other therapeutic interventions and/or recovery of the cardiac function.

In all these cases, it is reasonable to envisage, also on the basis of scientific evidence that has emerged over the years, that the pulsatility of perfusion will have favourable effects but it is also necessary to pay attention in order to prevent the pulsatility of artificial perfusion from being in opposition with that of the natural heart. For this purpose, the situation, defined as counterpulsation, in which the pressure wave generated by the system of assistance acts in the aorta during the diastole phase of the natural heart is considered optimal. In this case, in fact, the work done by the natural heart is minimized, and coronary perfusion is maximized.

To be able to operate in this mode, a system of modulation of the blood flow as the one described herein receives (through the unit 6, usually already configured for this function) an external synchronization signal, typically an ECG trace.

From this signal the control unit 6 can readily obtain not only the frequency of actuation desired but also the timing for the start of the phases of systole $T_s$ and of diastole $T_d$. This can occur, for example, by adopting the diagram shown in Figure 8. In said figure the references Tn, Tn+1, Tn+2, ... designate the periods between the successive QRS signals of ECG trace while, in the lower part of the figure, the systole S and the diastole D are shown both for the natural heart NH and for the pulsator P. It is to be borne in mind that the sum of the two coefficients of proportionality $\alpha$ and $\beta$ indicated in the figure is smaller than 1, for example $\alpha = 0.5$ and $\beta = 0.4$.

Also in this case the system to operate in "full fill - partially empty" mode.

This condition can be reached and maintained, operating as follows:

- starting the system by synchronizing the start of systole and the start of diastole with the external signal (ECG); monitoring, in the course of the diastole $T_d$, the current to the motor 4a of the pump 4 or the pressure in the gap (chambers 14) of the pulsator 1, interrupting the action of suction of the pump 4 when these signals indicate that the pulsator 1 is full; operating the pump, in the systole phase $T_s$, with a compression pulse having a form as described previously, duration equal to the one indicated by the external signal, and minimum amplitude. In these conditions, the pulsator always remains full, behaving substantially as a tube, and the flow remains basically constant;

- increasing progressively the amplitude of the compression pulse applied in the course of the systole $T_s$; when this reaches an adequate value, the pulsator 1 starts to modulate the flow emptying a little, and then filling up again in the course of diastole; monitoring, in the course of diastole, the outgoing flow, verifying the duration of the period during which the action of suction of the pump manages to maintain this flow practically zero;

- continuing to increase slowly the amplitude of the compression pulse applied in the course of systole, thus increasing the amplitude of the modulation of flow, until it reaches the instant in which there is complete filling of the pulsator near the end of the diastole phase programmed (e.g., at 80-90% of the duration set for the diastole);

- once this condition has been reached, activating a control of the amplitude of the systole pulse that will maintain the duration of the phase of filling of the pulsator equal to the desired fraction (e.g., 80-90%, and hence a substantial fraction) of the duration of the diastole, bearing in mind that, if the pulsator finishes filling first., it means that, in the course of the previous systole, it had emptied less than was desired (ejection volume smaller than the optimal) and hence it is necessary to increase a little the amplitude of the systole pulse; instead, if the time for filling is greater than what is desired, this means that with the previous systole the pulsator had emptied excessively and hence the amplitude of the systole pulse must be reduced: basically, the approach is to operate by increasing and decreasing the intensity of the transfer of the actuation fluid to the actuation chambers 14 in the course of systole according to whether, respectively, the phase of maximum extension of the cross section and hence of complete filling of the duct 10 tends to be anticipate or to be delayed.

Of course, without prejudice to the principle of the invention, the details of implementation and the embodiments may vary widely with respect to what is described and illustrated herein, without thereby departing from the scope of the present invention, as defined by the annexed claims. This applies, for example, to applications different from the systems of extracorporeal circulation and to devices of cardiac assistance, such as the so-called bioreactors for cell growth and other applications in which it is desired to have available pulsatile flows.

## Claims

1. A pulsator device (1) comprising a body that is able to define a deformable duct (10) for passage of a fluid with substantially constant incoming flow ($Q_0$), said deformable duct (10) having associated at least one external actuation chamber (14) that is selectively expandable with deformation of said duct (10) between at least one contracted condition and at least one extended condition so as to produce a variation in the volume of said duct (10) for passage of said fluid; the variation of said volume causing a modulation of the outgoing flow from the device and generation of a pulsed flow ($Q(t)$) of said fluid which traverses the duct.

2. The device according to Claim 1, **characterized in that** it is without valve elements for regulation of the flow of said fluid that traverses it.

3. The device according to Claim 1 or Claim 2, **characterized in that** said duct (10) has a tubular shape.

4. The device according to any one of the preceding claims, **characterized in that** said deformable duct (10) is constituted, at least in a position corresponding to said at least one actuation chamber (14), by material chosen from the group constituted by silicone rubber, polyurethane, polyvinyl chloride, nylon or pebax.

5. The device according to any one of the preceding claims, **characterized in that** said at least one actuation chamber (14) has a cross section which is as a whole arched.

6. The device according to any one of the preceding claims, **characterized in that** said at least one actuation chamber (14) extends in a substantially continuous way throughout the entire longitudinal development of said duct (10).

7. The device according to any one of the preceding claims, **characterized in that** it comprises a plurality of said actuation chambers (14), distributed angularly about said duct (10).

8. The device according to Claim 7, **characterized in that** it comprises three actuation chambers (14) distributed angularly about said duct (10).

9. The device according to Claim 7 or Claim 8, **characterized in that** said actuation chambers (14) have an angular extension of just less than 120°.

10. The device according to any one of the preceding claims, **characterized in that** it comprises a containment shell (12) for said deformable duct (10), said containment shell (12) being substantially rigid and defining with respect to said deformable duct (10) at least one gap defining said at least one selectively expandable actuation chamber (14).

11. The device according to any one of the preceding claims, **characterized in that** it has associated thereto a flowmeter (3) for measuring the flow ($Q(t)$) of said fluid coming out of the device itself.

12. The device according to Claim 11, **characterized in that** said flowmeter (3) is based upon a sensor without contact with said fluid subjected to pumping.

13. The device according to any one of the preceding claims, **characterized in that** it has associated thereto a pump (4) for transferring an actuation fluid with respect to said at least one selectively expandable actuation chamber (14).

14. The device according to any one of the preceding claims, **characterized in that** it has associated thereto a pressure meter (7) sensitive to the pressure in said at least one actuation chamber (14).

15. The device according to any one of the preceding claims, **characterized in that** it has associated thereto a system for monitoring and/or regulation of the volume of an actuation fluid fed into said at least one actuation chamber (14).

16. A method for operating a pulsator device (1) comprising a body defining a deformable duct (10) for passage of a fluid that enters the device with a substantially constant flow ($Q_0$), said duct (10) having associated thereto at least one actuation chamber (14) that is selectively expandable between at least one contracted condition and at least one extended condition so as to produce a variation in the volume of said duct (10) for passage of said fluid; the method comprising the operation of transferring (4, 5) in a controlled way (6) an actuation fluid with respect to said at least one actuation chamber (14), causing, as a result of the passage of said at least one actuation chamber (14) between said at least one contracted resting condition and at least one extended condition and of the consequent variation in said volume of the duct (10), modulation of the outgoing flow and generation of a pulsed flow (Q(t)) of said fluid.

17. The method according to Claim 16, **characterized in that** said operation of transferring (4, 5) in a controlled way (6) an actuation fluid with respect to said at least one actuation chamber (14) is conducted by modifying selectively the volume of said duct (10) in alternating phases of systole ($T_s$) and diastole ($T_d$).

18. The method according to Claim 17, **characterized in that** it comprises the operation of increasing, during said systole phase, the volume of the actuation chamber (14) in such a way as to reduce the volume of the duct (10) and to generate a pulse in the flow coming out of the device.

19. The method according to either Claim 17 or Claim 18, **characterized in that** it comprises the operation of rendering the value of said pulsed flow (Q(t)) of said fluid subjected to pumping substantially equal to zero for at least one first part of said diastole phase (D) and equal to the incoming flow ($Q_0$) during the possible residual part of the diastole phase.

20. The method according to any one of Claims 17 to 19, **characterized in that** it comprises the operation of bringing said duct (10), in the course or at the end of said diastole phase $T_d$, into a position of complete expansion of the section of the duct for passage of said flow.

21. The method according to any one of Claims 16 to 20, **characterized in that** it comprises the operation of using, as actuation fluid transferred with respect to said at least one actuation chamber (14), a liquid or a gas.

22. The method according to any one of Claims 17 to 21, **characterized in that** it comprises the operation of actuating said pulsator device (1) between said alternating phases of systole ($T_s$) and diastole ($T_d$) according to an operating mode chosen from:

   - asynchronous pulsation with fixed ejection volume;
   - asynchronous pulsation at a predetermined frequency; and
   - pulsation synchronous with a reference signal.

23. The method according to Claim 22, **characterized in that**, in said operating mode with asynchronous pulsation with fixed ejection volume, it comprises the operation of transferring (4, 5) said actuation fluid to said at least one actuation chamber (14) in such a way as to maintain the flow leaving the device in the course of the diastole phase and in the course of the systole phase, following a law of a predetermined form, with controlled amplitude and duration (6), such as to cause an ejection volume per cycle equal to the pre-set one.

24. The method according to either Claim 22 or Claim 23, **characterized in that**, in said operating mode with asynchronous pulsation with fixed ejection volume, it comprises the operation of varying the duration of the systole phase ($T_s$) to keep it proportional (for example equal) to the duration of the diastole phase ($T_d$).

25. The method according to any one of Claims 22 to 24, **characterized in that**, in said operating mode with asynchronous pulsation with fixed ejection volume, it comprises the operation of producing, as a result of the passage of said at least one actuation chamber (14) between said at least one contracted resting condition and at least one extended condition, a variation in said section for passage of said duct (10) with a range close to the maximum range allowed by said duct (10) between a condition of maximum extension and a condition of maximum contraction.

26. The method according to any one of Claims 22 to 25, **characterized in that**, in said operating mode with asynchronous pulsation with fixed ejection volume, it comprises the operation of maintaining substantially zero the value of said pulsed flow (Q(t)) in said diastole phase ($T_d$) up to complete extension of the section for passage of said duct (10), performing said systole phase ($T_s$) up to ejection by the pulsator (1) of said desired fixed ejection volume.

27. The method according to Claim 21, **characterized in that**, in said operating mode with asynchronous pulsation at a predetermined frequency, it comprises the operation of transferring (4, 5) said actuation fluid to said at least one actuation chamber (14) with a variable law, varying said ejection volume and maintaining the frequency of said phases of systole ($T_s$) and diastole ($T_d$).

28. The method according to any one of Claims 22 or 27, **characterized in that**, in said operating mode with asynchronous pulsation at a predetermined frequency, it comprises the operation of producing, as a result of the passage of said at least one actuation chamber (14) between said at least one contracted resting condition and at least one extended condition of pumping, a variation of said volume of said duct (10) between a condition of maximum extension and a condition of partial contraction.

29. The method according to Claim 22, **characterized in that**, in said operating mode with pulsation synchronous with a reference signal, it comprises the operation of synchronizing with said reference signal the start of said phases of systole ($T_s$) and of diastole ($T_d$).

30. The method according to one of Claims 22 or 29, **characterized in that**, in said operating mode with pulsation synchronous with a reference signal, it comprises the operation of producing, as a result of the passage of said at least one actuation chamber (14) between said at least one contracted resting condition and at least one extended condition of pumping, a variation of said volume of said duct (10) between a condition of maximum extension and a condition of partial contraction.

31. The method according to either Claim 29 or Claim 30, **characterized in that** it comprises the operations of:

- a) synchronizing with said reference signal the start of said phases of systole ($T_s$) and of diastole ($T_d$) as well as the duration of said systole phase ($T_s$), determined by said reference signal, transferring (4, 5) during said systole phase a quantity of actuation fluid to said at least one actuation chamber with an action of minimum intensity, so that said duct (10) preserves substantially said condition of maximum expansion of the section of passage;
- b) transferring (4, 5) said actuation fluid to said at least one actuation chamber (14) with a progressively increasing intensity during said systole phase ($T_s$), so that the pulsator (1) starts to modulate said pulsed flow ($Q(t)$), monitoring, during said diastole phase ($T_d$) said pulsed flow ($Q(t)$), verifying the duration of the period during which the pulsator maintains the value of said pulsed flow ($Q(t)$) substantially zero;
- c) transferring (4, 5) said actuation fluid to said at least one actuation chamber (14) with intensity further increasing during said systole phase ($T_s$), until the instant of reaching said condition of maximum extension of the section of said duct (10) is brought into the proximity of the end of the diastole phase ($T_d$) determined by said reference signal; and
- d) once the condition referred to in point c) has been reached, transferring (4, 5) said actuation fluid to said at least one actuation chamber (14) so as to maintain the reaching of the maximum extension of the section of said duct (10) at a substantial fraction of the duration of said diastole phase ($T_d$), increasing and decreasing the intensity of transfer (4, 5) of said actuation fluid to said at least one actuation chamber (14) according to whether said instant of reaching said maximum extension of the section of said duct (10) tends to be anticipated or delayed, respectively.

32. A control system (6) configured for implementing the process according to any one of Claims 16 to 31.

33. A computer-program product, which can be loaded into the memory of at least one computer and comprises portions of software code for implementing the method according to any one of Claims 16 to 31.

Fig. 1

# Fig.2

# Fig.3

# Fig.4

$$Q_0 \longrightarrow \qquad Q(t) = Q_0 + dV(t)/dt$$

$$V = V(t)$$

EP 1 674 119 A1

# Fig. 5

a)

b)

# Fig. 6

a)

b)

# Fig. 7

a)

$T_S$  $T_d$  $T_S$  $T$

$t$

b)

$Q(t)$

$Q_0$

$T$  $T_d$  $T_S$

$t$

# Fig.8

**European Patent Office**

**EUROPEAN SEARCH REPORT**

Application Number

EP 04 42 5940

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 4 552 552 A (POLASCHEGG ET AL) 12 November 1985 (1985-11-12) <br><br> * the whole document * <br> ----- | 1-6, 10-18, 21,22, 32,33 | A61M1/10 |
| X | US 4 080 958 A (BREGMAN ET AL) 28 March 1978 (1978-03-28) * the whole document * ----- | 1-6,10, 16-33 | |
| A | US 4 782 817 A (SINGH ET AL) 8 November 1988 (1988-11-08) * the whole document * ----- | 1,16,32, 33 | |

TECHNICAL FIELDS SEARCHED (Int.Cl.7)

A61M

The present search report has been drawn up for all claims

| Place of search | Date of completion of the search | Examiner |
|---|---|---|
| Munich | 28 April 2005 | Borowski, A |

**ANNEX TO THE EUROPEAN SEARCH REPORT
ON EUROPEAN PATENT APPLICATION NO.**

EP 04 42 5940

28-04-2005

| Patent document cited in search report | | Publication date | Patent family member(s) | | Publication date |
|---|---|---|---|---|---|
| US 4552552 | A | 12-11-1985 | DE | 3205449 A1 | 01-09-1983 |
| | | | EP | 0088900 A2 | 21-09-1983 |
| US 4080958 | A | 28-03-1978 | DE | 2707951 A1 | 01-09-1977 |
| | | | FR | 2342078 A1 | 23-09-1977 |
| | | | GB | 1526693 A | 27-09-1978 |
| | | | JP | 52105695 A | 05-09-1977 |
| US 4782817 | A | 08-11-1988 | JP | 1052472 A | 28-02-1989 |
| | | | JP | 1765999 C | 11-06-1993 |
| | | | JP | 4050831 B | 17-08-1992 |